# EUROPÄISCHE PATENTSCHRIFT

(11) **EP 0 588 224 B1**
(45) Veröffentlichungstag und Bekanntmachung des Hinweises auf die Patenterteilung: **18.12.1996**
(21) Anmeldenummer: 93114393.7
(22) Anmeldetag: 08.09.1993
(51) Int. Cl.: C07D 307/32

(54) **Verfahren zur Herstellung von 3-(2'-Oxyethyl)-dihydro-2-(3H)furanonen**
Process for the preparation of 3-(2'-oxyethyl)-dihydro-2-(3H)furanones
Procédé de préparation de 3-(2'-oxyéthyl)-dihydro-2-(3H)-furanones

(30) Priorität: 18.09.1992 DE 4231297
(43) Veröffentlichungstag der Anmeldung: 23.03.1994
(73) Patentinhaber: BASF Aktiengesellschaft, 67063 Ludwigshafen (DE)
(72) Erfinder: Kuekenhoehner, Thomas, Dr., D-6737 Boehl-Iggelheim (DE); Goetz, Norbert, Dr., D-6520 Worms 1 (DE); Fischer, Rolf, Dr., D-6900 Heidelberg (DE); Schnurr, Werner, Dr., D-6719 Herxheim (DE); Borchers, Dirk, Dr., D-6701 Birkenheide (DE)

(56) Entgegenhaltungen:
- EP-A- 0 194 144
- EP-A- 0 246 581
- US-A- 2 443 827
- CHEMICAL ABSTRACTS, vol. 35, no. 5, 10. März 1941, Columbus, Ohio, US; abstract no. 1382.5, K.G. PAKENDORF 'A new type reaction of ethylene oxide. II. By-products from the condensation of ethylene oxide with malonic ester.'
- CHEMICAL ABSTRACTS, vol. 35, no. 11, 10. Juni 1941, Columbus, Ohio, US; abstract no. 3627.9, K.G. PAGENDORF ET AL. 'A new reaction of ethylene oxide; condensation of ethylene oxide with acetoacetate.'
- HELVETICA CHIMICA ACTA. Bd. 35, Nr. 7 , 1. Dezember 1952 , BASEL CH Seiten 2401 - 2406 L. WILLIMANN ET AL. 'Über 7-Methyl-octadien-(2,6)-ol-(1), ein "Apogeraniol"'

## Beschreibung

Die Erfindung betrifft ein verbessertes Verfahren zur Herstellung von 3-(2'-Oxyethyl)-dihydro-2(3H)furanonen durch Umsetzung von Ethylenoxid mit Acetessigsäureestern unter Alkalimetallalkoholat-Katalyse.

Aus Dokl. Akad. Nauk SSSR 27, 956 bis 959 (1940) ist die Herstellung von 3-(2'-Acetoxyethyl)-dihydro-2(3H)furanon durch Acylierung von 3-(2'-Hydroxyethyl)-dihydro-2(3H)furanon mit Acetanhydrid bekannt.

Aus Dokl. Akad. Nauk SSSR 29, 579 bis 581 (1940) ist die Herstellung von 3-(2'-Hydroxyethyl)-dihydro-2(3H)furanon durch Umsetzung von Acetessigsäureethylester mit Ethylenoxid in Gegenwart von katalytischen Mengen einer schwachen Basen wie z.B. Piperidin bei Raumtemperatur innerhalb von 20 Tagen bekannt. Derart lange Reaktionszeiten sind technisch ungeeignet. Ferner reagiert Ethylenoxid mit diesen Katalysatoren zu quartären Ammoniumsalzen, die bei der Aufarbeitung stören.

Ferner ist aus der US-A-4 831 166 die Verwendung anderer schwacher Basen sowie die katalytische Wirkung von neutralen Salzen, wie Alkalimetallhalogeniden, Ammoniumhalogeniden, Phosphoniumhalogeniden Alkaliphosphaten oder Alkalimetallcarbonaten, bei der Umsetzung von Acetessigsäurealkylestern mit Ethylenoxid zu 3-(2'-Hydroxyethyl)-dihydro-2(3H)furanon bekannt. Hierbei wird - je nach Art und Menge des verwendeten Lösungsmittels - ein Gemisch aus 3-(2'-Hydroxyethyl)-dihydro-2(3H)furanon und 3-(2'-Acetoxyethyl)-dihydro-2(3H)furanon erhalten. Für eine technische Anwendung sind die beschriebenen Katalysatoren jedoch nur bedingt geeignet, da zahlreiche der beschriebenen Katalysatoren wie z.B. Natriumchlorid, Alkaliphosphate oder Alkalicarbonate in den verwendeten Lösungsmitteln nicht löslich sind, so daß die Reaktion in einer heterogenen Mischung durchgeführt werden muß; zum anderen entstehen bei Verwendung von Halogenidsalzen aus den Halogenidionen und Ethylenoxid geringe Mengen von 2-Halogenethanolen (Liebigs Ann., 116, 252 (1860); Angew. Chem. 84,, 1173 (1972)), die sehr toxische Eigenschaften haben und sorgfältig, mit hohem Aufwand, von dem Produkt abgetrennt werden müssen, da sie bei vielen Folgereaktionen wie z.B. bei katalytischen Esterhydrierungen mit Wasserstoff aufgrund ihres Halogengehaltes schon in ppm-Mengen zu Katalysatorvergiftungen führen.

Z.B. aus Helv. Chim. Acta 35, 2401 (1952), EP-A-348 549, US-A-2 433 827 und CA-A-546 573 sind Reaktionen von Acetessigsäureestern mit Ethylenoxid unter Verwendung von katalytischen oder stöchiometrischen Mengen starker Basen, wie z.B. Natriummethylat oder Natronlauge, bei Temperaturen von (-10) bis + 40°C bei langen Reaktionszeiten bekannt, wobei als Produkt das 3-Acetyldihydro-2(3H)furanon, also das Additionsprodukt von einem Equivalent Ethylenoxid an den Acetessigester beschrieben wird.

Ferner ist aus Khim.-Farm. Zh., 23 (1), 70 (1989) die Zersetzung von 3-Acetyldihydro-2(3H)furanon durch alkoholische Alkoholat-Lösungen bekannt.

Der vorliegenden Erfindung lag daher die Aufgabe zugrunde, den zuvor genannten Nachteilen abzuhelfen.

Demgemäß wurde ein neues und verbessertes Verfahren zur Herstellung von 3-(2'-Oxyethyl)-dihydro-2(3H)furanonen der allgemeinen Formel I 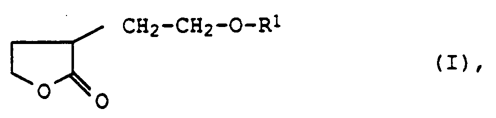 in der R¹ Wasserstoff oder Acetyl 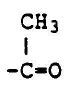 bedeutet, durch Umsetzung von Ethylenoxid mit Acetessigsäure estern der allgemeinen Formel II 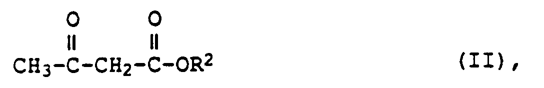 in der R² C₁- bis C₄-Alkyl bedeutet, gefunden, welches dadurch gekennzeichnet ist, daß man die Umsetzung in alkoholischen Lösungen in Gegenwart von Alkalimetallalkoholaten bei Temperaturen von 20 bis 100°C und Drücken von 1 bis 20 bar durchführt.

Das erfindungsgemäße Verfahren läßt sich wie folgt durchführen:

Zur Herstellung der 3-(2'-Oxyethyl)-dihydro-2(3H)furanone I legt man in der Regel den Acetessigsäureester II in alkoholischer Lösung in Gegenwart eines Alkalimetallalkoholates bei Temperaturen von 20 bis 100°C, bevorzugt 40 bis 80°C, besonders bevorzugt 50 bis 70°C und Drücken von 1 bis 20 bar, bevorzugt 1,5 bis 10 bar, besonders bevorzugt 2 bis 5 bar vor und dosiert dieser Mischung unter intensivem Rühren das - in der Regel - flüssige Ethylenoxid zu.

Es kann vorteilhaft sein, die Reaktion mit einem Temperaturgradienten durchzuführen. Beispielsweise kann während der Zudosierung des Ethylenoxides bei niedrigen Temperaturen von 40 bis 60°C und anschließend zur Vervollständigung der Reaktion bei höheren Temperatur von 60 bis 80°C gearbeitet werden.

Die Reaktion wird üblicherweise in geschlossenen Rührreaktoren durchgeführt, da einige der Reaktionskomponenten - insbesondere Ethylenoxid - bei den Reaktionstemperaturen teilweise gasförmig sind und sich nur zum Teil in der Reaktionsmischung lösen. In dem Reaktor stellt sich daher in der Regel ein Überdruck ein, der in etwa den Partialdampfdrücken der Reaktionskomponenten entspricht. Der Druck ist ferner abhängig von der Reaktionstemperatur.

Das Molverhältnis von Ethylenoxid zum Acetessigsäureester II beträgt in der Regel 3 : 1 bis 1 : 1, bevorzugt 2,2 : 1 bis 1,5 : 1, besonders bevorzugt 2 : 1.

Als Acetessigsäureester II eignen sich prinzipiell Ester beliebiger Alkohole wie C₁- bis C₂₀-Alkohole. In der Regel verwendet man Ester der C₁- bis C₄-Alkohole, bevorzugt Acetessigsäuremethylester und Acetessigsäureethylester, besonders bevorzugt Acetessigsäuremethylester.

Als Alkalimetallalkoholate eignen sich Lithium-, Natrium-, Kalium-, Rubidium- und Cäsiumalkoholate, bevorzugt Lithium-, Natrium- und Kaliumalkoholate beliebiger Alkohole wie C₁- bis C₂₀-Alkohole. In der Regel verwendet man Alkalimetallalkoholate von C₁- bis C₄-Alkoholen wie Natriummethylat, Natriumethylat, Kaliummethylat, Kaliumethylat und Kalium-tert.-butylat, bevorzugt Alkalimetallalkoholate von C₁- und C₂-Alkoholen wie Natriummethylat, Natriumethylat, Kaliummethylat und Kaliumethylat, besonders bevorzugt Natriummethylat.

Das Molverhältnis von Alkalimetallalkoholat zum Acetessigsäureester II beträgt in der Regel 0,001 : 1 bis 1 : 1, bevorzugt 0,01 : 1 bis 0,5 : 1, besonders bevorzugt 0,02 : 1 bis 0,2 : 1.

Die Alkalimetallalkoholate können als Feststoff in die Reaktion eingesetzt werden. Aus verfahrenstechnischen Gründen ist es jedoch vorteilhaft, mit Lösungen der Alkalimetallalkoholate zu arbeiten. Verwendung finden können im Prinzip alle Lösungsmittel, die gegen die verwendeten Alkoholate und unter den Reaktionsbedingungen stabil sind. Als besonders vorteilhaft erweist sich eine alkoholische Lösung der Alkalimetallalkoholate, da diese Lösungen einfach hergestellt werden können, z.B. durch Auflösen der jeweiligen Alkalimetalle in dem betreffenden Alkohol. Besonders bevorzugt ist eine Lösung von Natriummethylat in Methanol.

Als Lösungsmittel eignen sich alle gängigen Alkohole wie C₁- bis C₂₀-Alkohole, bevorzugt C₁- bis C₄-Alkohole wie Methanol, Ethanol, Propanol, iso-Propanol, Butanol, sek-Butanol, iso-Butanol und tert- Butanol, besonders bevorzugt Methanol und Ethanol, dabei ist es zum Erreichen guter Reaktionsergebnisse nicht notwendig, daß Lösungsmittel, Alkoholkomponente der Estergruppe im Acetessigester II und Alkoholkomponente des Alkalimetallalkoholat-Katalysators identisch sind.

Die Lösungsmittelmenge kann in weiten Bereichen gewählt werden, sie liegt jedoch zweckmäßigerweise bei 25 bis 500 ml, bevorzugt bei 50 bis 200 ml Alkohol pro Mol Acetessigester II.

Unter den alkalischen Reaktionsbedingungen finden üblicherweise Umesterungsreaktionen statt. Deshalb wird in der Regel ein Gemisch aus 3-(2'-Acetoxyethyl)-dihydro-2(3H)furanon und 3-(2'-Hydroxyethyl)- dihydro-2(3H)furanon, das als Folgeprodukt von 3-(2'-Acetoxyethyl)-dihydro-2(3H)furanon durch Methanolyse unter den alkalischen Reaktionsbedingungen entsteht, erhalten. Durch Variierung der bei der Reaktion als Lösungsmittel verwendeten Alkoholmenge läßt sich das Verhältnis von 3-(2'-Hydroxyethyl)-dihydro-2(3H)furanon und 3-(2'-Acetoxyethyl)-dihydro-2(3H)furanon, das in etwa dem Veresterungsgleichgewicht entspricht, steuern.

Durch Nachbehandlung mit geringen Mengen eines Alkalimetallalkoholates in z.B. alkoholischer Lösung kann das Gleichgewicht in Richtung des 3-(2'-Hydroxyethyl)-dihydro-2(3H)furanon verschoben werden. Durch Veresterung mit z.B. Acetanhydrid oder Essigsäure, gegebenenfalls in Gegenwart eines Katalysators, z.B. einer starken Säure wie Schwefelsäure, Phosphorsäure, einer organische Sulfonsäuren wie Methansulfonsäure, Benzolsulfonsäure, Toluolsulfonsäure oder eines sauren Ionenaustauschers, läßt sich das Gemisch selektiv in das 3-(2'-Acetoxyethyl)-dihydro-2(3H) furanon überführen.

Das erfindungsgemäße Verfahren läßt sich mit den üblichen Verfahrenstechniken z.B. in Rohrreaktoren oder Rührkesselkaskaden, diskontinuierlich oder kontinuierlich betreiben. Der Katalysator, das Alkali- metallalkoholat, kann durch Neutralisation mit einer Säure, z.B. Essigsäure, Schwefelsäure oder Phosphorsäure, anschließender Abfiltration der ausgefallenen Salze aus dem Reaktionsgemisch entfernt werden. Dies erleichtert die weitere Aufarbeitung nach üblichen Verfahren wie Destillation oder Extraktion.

Soll eine wäßrige Aufarbeitung erfolgen, so empfiehlt es sich, das Reaktionsgemisch aus 3-(2'-Acetoxyethyl)-dihydro-2(3H)furanon und 3-(2'-Hydroxyethyl)-dihydro-2(3H)furanon zu verestern (zu acylieren), da sich 3-(2'-Acetoxyethyl)-dihydro-2(3H)furanon mit organischen Lösungsmitteln leichter extrahieren läßt.

Die Acetessigsäureester II und die Alkalimetallalkoholate sind entweder bekannt oder können leicht nach bekannten Verfahren hergestellt werden.

Die 3-(2'-Oxyethyl)-dihydro-2(3H)furanone I sind vielseitig verwendbare Verbindungen, zum Beispiel für Riechstoffe, Pharmazeutika oder aufgrund ihrer Polyfunktionalität als Vorprodukte für Polymere. Besondere Bedeutung kommt ihnen als Ausgangsverbindungen für die Synthese von Pestiziden zu. Beispielsweise können 3-(2'-Hydroxyethyl)-dihydro-2(3H)furanon und 3-(2'-Acetoxyethyl)-dihydro-2(3H)furanon nach den in der EP-A-284 969 bzw. der US-A-4 837 346 beschriebenen Verfahren zu Tetrahydropyran-4-carbonsäureestern umgewandelt werden, die ihrerseits wichtige Zwischenprodukte zur Herstellung von Pflanzenschutzmitteln, wie sie z.B. in EP-A-142 741 beschrieben werden, sind.

### Beispiele

### Beispiel 1

In einem 3,5 l-Druckbehälter wurde eine Mischung aus 696 g (6 Mol) Acetessigsäuremethylester, 840 ml Methanol und 108 g einer 30 gew.-%igen Natriummethylat-Lösung in Methanol (0,6 Mol) vorgelegt, 264 g (6,0 Mol) Ethylenoxid bei 60°C unter kräftigem Rühren innerhalb 3 Stunden zugepumpt und anschließend 24 Stunden bei 60°C nachgerührt. Der Reaktionsaustrag wurde mehrfach mit Stickstoff gespült und anschließend der Katalysator mit 42 g (0,7 Mol) Essigsäure neutralisiert. Die niedrig siedenden Anteile wurden bis zu einer Übergangstemperatur von 55°C bei 120 mbar abdestilliert, und bei einer Übergangstemperatur bis zu 78°C bei 2 mbar wurden 252 g (2,2 Mol) Acetessig- säuremethylester als Zwischensieder-Fraktion zurückgewonnen. Dem Destillationsrückstand wurden bei 110°C innerhalb einer Stunde 714 g (7 Mol) Acetanhydrid zugetropft, eine Stunde nachgerührt und überschüssiges Acetanhydrid sowie die entstandene Essigsäure abdestilliert. Der Rückstand wurde fraktioniert. Man erhielt 23 g (0,2 Mol) 2-Acetylbutyrolacton (Sdp.: 120°C/13 mbar) und 304 g (1,8 Mol) 3-(2'-Acetoxyethyl)-dihydro-2(3H)furanon (Sdp.: 135°C/3 mbar).

### Beispiel 2

In einem 3,5 l-Druckbehälter wurde eine Mischung aus 696 g (6 Mol) Acetessigsäuremethylester, 840 ml Methanol und 108 g einer 30 gew.-%igen Natriummethylat-Lösung in Methanol (0,6 Mol) vorgelegt, 528 g (12,0 Mol) Ethylenoxid bei 60°C unter kräftigem Rühren innerhalb 3 Stunden zugepumpt und anschließend 24 Stunden bei 60°C nachgerührt. Der Reaktionsaustrag wurde mehrfach mit Stickstoff gespült und anschließend der Katalysator mit 42 g (0,7 Mol) Essigsäure neutralisiert. Die niedrig siedenden Anteile wurden bis zu einer Übergangstemperatur von 80°C bei 200 mbar abdestilliert. Dem Destillationsrückstand wurden bei 110°C innerhalb einer Stunde 714 g (7 Mol) Acetanhydrid zugetropft, eine Stunde nachgerührt, überschüssiges Acetanhydrid sowie die entstandene Essigsäure abdestilliert und der Rohaustrag abgekühlt. Nach Zugabe von 665 g Toluol und 200 g Wasser wurden die Phasen getrennt und die wäßrige Phase einmal mit 100 ml Toluol extrahiert. Nach Entfernung des Lösungsmittels und Rektifikation erhielt man 742 g (72 %) 3-(2'-Acetoxyethyl)-dihydro-2(3H)furanon; Sdp.: 135°C/3 mbar.

### Beispiel 3

In einem 3,5 l-Druckbehälter wurde eine Mischung aus 696 g (6 Mol) Acetessigsäuremethylester, 840 ml Methanol und 216 g einer 30 gew.-%igen Natriummethylat-Lösung in Methanol (1,2 Mol) vorgelegt, 528 g (12,0 Mol) Ethylenoxid bei 40°C unter kräftigem Rühren innerhalb 1,5 Stunden zugepumpt und 16 Stunden bei 50°C nachgerührt. Die Aufarbeitung wurde wie in Beispiel 2 durchgeführt. Man erhielt 692 g (67 %) 3-(2'-Acetoxyethyl)-dihydro-2(3H)furanon.

### Beispiel 4

In einem 3,5 l-Druckbehälter wurde eine Mischung aus 696 g (6 Mol) Acetessigsäuremethylester, 840 ml Methanol und 108 g einer 30 gew.-%igen Natriummethylat-Lösung in Methanol (0,6 Mol) vorgelegt, 528 g (12 Mol) Ethylenoxid bei 40°C unter kräftigem Rühren innerhalb 3 Stunden zugepumpt und 60 Stunden bei 40°C nachgerührt. Die Aufarbeitung wurde wie in Beispiel 2 durchgeführt. Man erhielt 782 g (76 %) 3-(2'-Acetoxyethyl)-dihydro-2(3H)furanon.

## Patentansprüche

1. Verfahren zur Herstellung von 3-(2'-Oxyethyl)-dihydro-2(3H)furanonen der allgemeinen Formel I 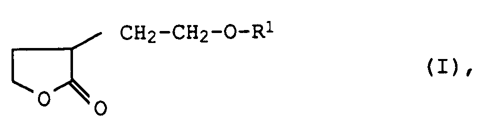 in der R¹ Wasserstoff oder Acetyl bedeutet, durch Umsetzung von Ethylenoxid mit Acetessigsäureestern der allgemeinen Formel II 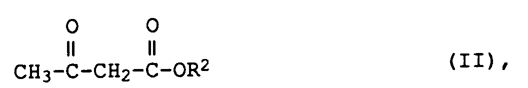 in der R² C₁- bis C₄-Alkyl bedeutet, dadurch gekennzeichnet, daß man die Umsetzung in alkoholischen Lösungen in Gegenwart von Alkalimetallalkoholaten bei Temperaturen von 20 bis 100°C und Drücken von 1 bis 20 bar durchführt.

2. Verfahren zur Herstellung von 3-(2'-Oxyethyl)-dihydro-2(3H)furanonen nach Anspruch 1, dadurch gekennzeichnet, daß man die Umsetzung im Molverhältnis von Alkalimetallalkoholat zum Acetessigsäureester II von 0,001 : 1 bis 1 : 1 durchführt.

3. Verfahren zur Herstellung von 3-(2'-Oxyethyl)-dihydro-2(3H)furanonen nach Anspruch 1, dadurch gekennzeichnet, daß man die Umsetzung im Molverhältnis von Alkalimetallalkoholat zum Acetessigsäureester II von 0,01 : 1 bis 0,5 : 1 durchführt.

4. Verfahren zur Herstellung von 3-(2'-Oxyethyl)-dihydro-2(3H)furanonen nach Anspruch 1, dadurch gekennzeichnet, daß man als Alkalimetallalkoholat Natrium- oder Kalium-C₁- bis C₄-alkoholate verwendet.

5. Verfahren zur Herstellung von 3-(2'-Oxyethyl)-dihydro-2(3H)furanonen nach Anspruch 1, dadurch gekennzeichnet, daß man als Alkalimetallalkoholat Natriummethylat, Kaliummethylat, Natriumethylat oder Kaliumethylat verwendet.

6. Verfahren zur Herstellung von 3-(2'-Oxyethyl)-dihydro-2(3H)furanonen nach Anspruch 1, dadurch gekennzeichnet, daß man als Alkalimetallalkoholat Natriummethylat verwendet.

7. Verfahren zur Herstellung von 3-(2'-Oxyethyl)-dihydro-2(3H)furanonen nach Anspruch 1, dadurch gekennzeichnet, daß man als Alkalimetallalkoholat Natriummethylat und als alkoholische Lösung eine methanolische Lösung verwendet.

8. Verfahren zur Herstellung von 3-(2'-Oxyethyl)-dihydro-2(3H)furanonen nach Anspruch 1, dadurch gekennzeichnet, daß man die Umsetzung bei Temperaturen von 40 bis 80°C durchführt.

## Claims

1. A process for preparing 3-(2'-oxyethyl)dihydro-2(3H)furanones of the general formula I 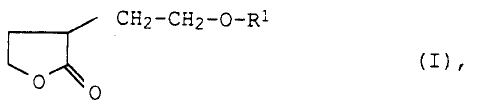 where R¹ is hydrogen or acetyl, by reacting ethylene oxide with acetoacetic acid esters of the general formula II 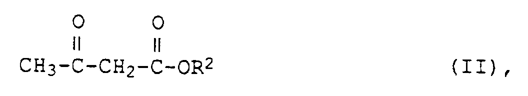 where R² is C₁-C₄-alkyl, which comprises carrying out the reaction in alcoholic solutions in the presence of alkali metal alkoxides at from 20 to 100°C and from 1 to 20 bar.

2. A process for preparing 3-(2'-oxyethyl)dihydro-2(3H)furanones as claimed in claim 1, wherein the reaction is carried out in a molar ratio of alkali metal alkoxide to the acetoacetic acid ester II of 0.001 : 1 - 1 : 1.

3. A process for preparing 3-(2'-oxyethyl)dihydro-2(3H)furanones as claimed in claim 1, wherein the reaction is carried out in a molar ratio of alkali metal alkoxide to the acetoacetic ester II of 0.01 : 1 - 0.5 : 1.

4. A process for preparing 3-(2'-oxyethyl)dihydro-2(3H)furanones as claimed in claim 1, wherein the alkali metal alkoxide used is sodium or potassium C₁-C₄-alkoxide.

5. A process for preparing 3-(2'-oxyethyl)dihydro-2(3H)furanones as claimed in claim 1, wherein the alkali metal alkoxide used is sodium methoxide, potassium methoxide, sodium ethoxide or potassium ethoxide.

6. A process for preparing 3-(2'-oxyethyl)dihydro-2(3H)furanones as claimed in claim 1, wherein the alkali metal alkoxide used is sodium methoxide.

7. A process for preparing 3-(2'-oxyethyl)dihydro-2(3H)furanones as claimed in claim 1, wherein the alkali metal alkoxide used is sodium methoxide and the alcoholic solution used is a methanolic solution.

8. A process for preparing 3-(2'-oxyethyl)dihydro-2(3H)furanones as claimed in claim 1, wherein the reaction is carried out at from 40 to 80°C.

## Revendications

1. Procédé de préparation de 3-(2'-oxyéthyl)-dihydro-2(3H)furannones répondant à la formule générale I 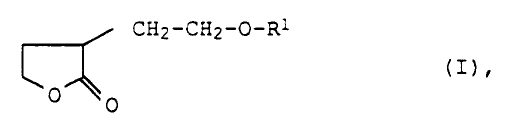 dans laquelle R¹ représente un atome d'hydrogène ou le radical acétyle, par la réaction de l'oxyde d'éthylène avec des esters de l'acide acétoacétique de la formule générale II 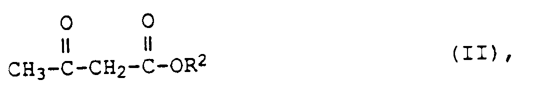 dans laquelle R² représente un radical alkyle en C₁ à C₄, caractérisé en ce que l'on entreprend la réaction en solutions alcooliques et en présence d'alcoolates de métaux alcalins à des températures de 20 à 100°C et sous de pressions de 1 à 20 bars.

2. Procédé de préparation de 3-(2'-oxyéthyl)-dihydro-2(3H)furannones suivant la revendication 1, caractérisé en ce que l'on entreprend la réaction dans le rapport molaire de l'alcoolate de métal alcalin à l'ester de l'acide acétoacétique II de 0,001:1 à 1:1.

3. Procédé de préparation de 3-(2'-oxyéthyl)-dihydro-2(3H)furannones suivant la revendication 1, caractérisé en ce que l'on entreprend la réaction dans le rapport molaire de l'alcoolate de métal alcalin à l'ester de l'acide acétoacétique de 0,01:1 à 0,5:1.

4. Procédé de préparation de 3-(2'-oxyéthyl)-dihydro-2(3H)furannones suivant la revendication 1, caractérisé en ce que l'on utilise, à titre d'alcoolate de métal alcalin, des alcoolates en C₁ à C₄ de sodium ou de potassium.

5. Procédé de préparation de 3-(2'-oxyéthyl)-dihydro-2(3H)furannones suivant la revendication 1, caractérisé en ce que l'on utilise, à titre d'alcoolate de métal alcalin, le méthylate de sodium, le méthylate de potassium, l'éthylate de sodium ou l'éthylate de potassium.

6. Procédé de préparation de 3-(2'-oxyéthyl)-dihydro-2(3H)furannones suivant la revendication 1, caractérisé en ce que l'on utilise, à titre d'alcoolate de métal alcalin, le méthylate de sodium.

7. Procédé de préparation de 3-(2'-oxyéthyl)-dihydro-2(3H)furannones suivant la revendication 1, caractérisé en ce que l'on utilise, à titre d'alcoolate de métal alcalin, le méthylate de sodium et, à titre de solution alcoolique, une solution méthanolique.

8. Procédé de préparation de 3-(2'-oxyéthyl)-dihydro-2(3H)furannones suivant la revendication 1, caractérisé en ce que l'on entreprend la réaction à des températures de 40 à 80°C.
